# FASCICULE DE BREVET EUROPEEN

(11) **EP 2 475 689 B1**
(45) Date de publication et mention de la délivrance du brevet: **26.03.2014**
(21) Numéro de dépôt: 10735256.9
(22) Date de dépôt: 19.07.2010
(51) Int. Cl.: C08F 6/28, C08F 8/12, C08F 8/50, C07C 67/03

(54) **PROCÉDÉ DE RECYCLAGE STÉRÉOSPÉCIFIQUE D'UN MÉLANGE DE POLYMÈRES À BASE DE PLA**
VERFAHREN FÜR STEREOSPEZIFISCHES RECYCLING EINER PLA-POLYMER-MISCHUNG
METHOD FOR STEREOSPECIFICALLY RECYCLING A PLA POLYMER MIXTURE

(30) Priorité: 10.09.2009 BE 200900554
(43) Date de publication de la demande: 18.07.2012
(73) Titulaire: Futerro S.A., 7760 Escanaffles (BE)
(72) Inventeur: COSZACH, Philippe, B-7760 Escanaffles (BE); WILLOCQ, Jonathan, B-7760 Escanaffles (BE)
(74) Mandataire: Leyder, Francis
(86) Numéro de dépôt international: PCT/EP2010/060426
(87) Numéro de publication internationale: WO 2011/029648

(56) Documents cités:
- EP-A1- 0 628 533
- WO-A1-92/00974

## Description

### Domaine de l'invention

La présente invention se rapporte à un procédé de recyclage chimique stéréospécifique d'un mélange de polymères à base de PLA, pour en reformer son monomère ou un des ses dérivés. Ces derniers pouvant entrer dans le marché traditionnel des lactates ou à nouveau servir comme matière première pour la synthèse de PLA.

A l'heure actuelle, afin de promouvoir l'essor des biopolymères, dont l'utilisation s'inscrit dans une perspective de respect de l'environnement, il est essentiel de pouvoir démontrer la viabilité de la gestion de la fin de vie de ces produits. L'objectif de la présente invention est de répondre à cette problématique pour le cas de l'acide polylactique (PLA) en proposant une solution originale se démarquant de celles déjà existantes de part son caractère stéréospécifique.

### Etat de l'art

La gestion de la fin de vie des matières plastiques est un élément très important dans la viabilité d'un plastique sur le marché (par exemple, le PVC a été retiré du marché des bouteilles en plastique faute d'un système de recyclage efficace). Tout comme les plastiques d'origine non renouvelable (issus de la pétrochimie) et bien que leurs filières de fin de vie soient plus nombreuses, les biopolymères sont confrontés à des défis techniques lorsqu'il s'agit de cette gestion de fin de vie. Notamment lorsque l'on parle de volumes très importants, générés dans les marchés de commodité. C'est pourquoi, il est important de traiter ce problème.

A l'heure actuelle, différentes voies permettant de gérer la fin de vie des déchets sont déjà connues tels que la mise en décharge, l'incinération, le compostage ou encore le recyclage mécanique.

Ces différentes techniques de fin de vie ne sont pas idéales car les matières plastiques ne sont pas recyclées en éléments de base (monomères) et donc directement et perpétuellement utilisables. Malgré tout, ces procédés sont viables pour le PLA mais cela uniquement si le flux de matière est composé exclusivement de PLA. En effet, si d'autres polymères contaminent le PLA, les différentes techniques précitées sont rendues difficiles. Par exemple dans le cas d'une contamination au PET, ce dernier n'est pas dégradé dans un composte. Dans le cas d'une contamination au PVC, une incinération est possible mais implique l'utilisation de filtres coûteux en raison des dégagements nocifs. Quant au recyclage mécanique, le produit obtenu est complètement dénaturé s'il est composé d'un mélange de polymères.

Le recyclage chimique est souvent cité comme la voie idéale de recyclage, il consiste à transformer le polymère par un processus chimique tel que par exemple : le craquage thermique ou catalytique en hydrocarbure, la pyrolyse qui redonne les monomères, ... Un système de recyclage chimique pour le PET est connu, il s'agit de sa dépolymérisation par un diol, appelé aussi glycolyse. La chaîne moléculaire est rompue et les produits obtenus sont de l'acide téréphtalique et de l'éthylène glycol. Néanmoins, certains mécanismes de dégradation lors de cette dépolymérisation, engendrent des modifications structurales irréversibles de la matière, modifications qui peuvent être responsables de difficultés lors de transformations successives.
Un système de recyclage chimique du PLA peut également être envisagé afin de récupérer le monomère, l'acide lactique ou l'un de ses dérivés. Certains brevets revendiquent par exemple, une hydrolyse (Brake L.D. , Subramanian N.S., U.S. Patent 5,229,528, 1993 ; Galactic, BE Patent 56,491, 2009) ou une solvolyse (Brake L.D., U.S. Patent 5,264,614, 1993 ; Brake L.D., U.S. Patent 5,264,617, 1993 ; Galactic, BE Patent 56,493, 2009) de poly(hydroxy-acide) incluant le PLA avec production d'hydroxy-acides ou de leurs esters. Des dégradations thermiques (pyrolyse par exemple) du PLA sont également connues menant à la formation de lactide (F.D. Kopinke, M. Remmler, K. Mackenzie, M. Möder, O. Wachsen, Polymer Degradation and stability, 53, 329-342, 1996) par un mécanisme de cyclisation par addition-élimination. Mais ces méthodes présentent un faible rendement en monomères. De plus, ces différentes techniques sont souvent réalisées à haute température et/ou haute pression ce qui provoque une dégradation chimique et optique de l'acide lactique obtenu.
Il est bien connu qu'il existe deux formes optiquement actives d'acide lactique : acide L-lactique (L-LA) et acide D-lactique (D-LA). Dès lors, le lactide, dimère cyclique de l'acide lactique, peut se présenter sous trois formes diastéréoisomériques selon qu'ils sont constitués de deux molécules d'acide D-lactique : D-lactide, de deux molécules d'acide L-lactique : L-lactide, ou d'une molécule d'acide L-lactique et d'une molécule D-lactique : méso-lactide. Signalons que le méso-lactide est caractérisé par un point de fusion aux alentours de 50°C alors que celles des isomères L- et D-lactide est de 97°C.
Une problématique commune à tous ces procédés est qu'aucun n'est stéréospécifique. En effet, ils ne permettent pas d'éliminer les énantiomères D pouvant être générés, d'une part, par l'ensemble du processus de production du PLA, et d'autre part, par les différent processus de recyclage chimique. Ce qui signifie la formation d'énantiomères D lorsque l'on travaille avec un PLA de type L (forme essentiellement présente sur le marché). De manière identique, il y aura formation d'énantiomères L lorsque l'on travaillera avec du PLA de type D.
Cet enrichissement progressif en énantiomère non désiré au niveau de l'acide lactique ou de ses dérivés, aura un impact important sur le rendement et les coûts de production d'un nouveau PLA.

Le document EP 0628533 décrit un procédé de recyclage d'une source contenant un polymère tel qu'un polylactide. Ledit procédé comprend une étape de solubilisation du polymère suivie d'une dépolymérisation. Pour ces étapes le solvant utilisé peut être un alcool comprenant de 1 à 6 atomes de carbones, auquel cas lorsque la température est par la suite poussée entre 100°C et 200°C une alcoolyse a lieu pour provoquer la dépolymérisation.

Il existe donc un besoin pour un procédé de recyclage stéréospécifique du PLA simple, efficace et non-dénaturant afin de reformer son monomère ou l'un de ses dérivés. Ces derniers pouvant entrer dans le marché traditionnel des lactates ou à nouveau servir comme matière première pour la synthèse de PLA.

Dans un but de simplification, nous présenterons dans la suite du présent document l'invention au départ d'un PLA de type L (majorité des éléments constitutifs de type L) sachant qu'elle pourra tout aussi bien convenir au départ d'un PLA de type D (majorité des éléments constitutifs de type D).

### Brève description de l'invention

La présente invention a pour objet un procédé de recyclage chimique et stéréospécifique d'un mélange de polymères contenant nécessairement du PLA, en lactide ou un de ses dérivés, pouvant soit être hydrolysé en acide lactique ou un de ses dérivés, soit être utilisé comme monomère ou co-monomère pour la synthèse de PLA ou de copolymères de PLA.

Ce procédé de recyclage chimique et stéréospécifique d'un mélange de polymères contenant du PLA est caractérisé en ce qu'il comprend:
a. une mise en solution du mélange de polymères dans un ester lactique apte à dissoudre la fraction PLA ;
b. une dépolymérisation par transestérification de la fraction de PLA provenant de l'étape précédente, cette dépolymérisation étant arrêtée lorsque le mélange d'oligoesters provenant de la dépolymérisation atteint une masse moléculaire moyenne en nombre comprise entre 400 et 5.000 uma.

Une forme de réalisation de la présente invention est qu'entre les étapes a et b, peut être comprise une étape de séparation de l'ester lactique et du PLA dissous d'une part, et du mélange de polymères non solubles d'autre part.

Une autre forme de réalisation de la présente invention consiste à soumettre le mélange d'oligoesters de l'étape b à une étape de cyclisation catalytique avec production d'une phase vapeur riche en lactide et une phase liquide riche en oligoesters.

Une autre forme de réalisation de la présente invention consiste à broyer et/ou compacter, au préalable de la mise en suspension, le mélange de polymères jusqu'à obtenir un rapport poids/volume compris entre 0,005 et 1, 4 t/m³.

Une autre forme de réalisation de la présente invention consiste à séparer l'ester lactique résiduel des oligoesters avant d'entamer l'étape de cyclisation.

Un autre objet de l'invention est de proposer un procédé de production de PLA plus simple et économique. En effet, contrairement aux procédés conventionnels au départ de l'acide lactique ou de l'un de ses dérivés où une purification poussée est opérée tant au niveau de la production de l'acide lactique que du lactide, dans le cadre de la présente invention, la purification n'est opérée qu'à une seule reprise au niveau de la production du lactide.

La présente invention a également pour objet la purification isomérique du flux de PLA traité par recyclage chimique et destiné à la production d'un nouveau PLA. En effet, le processus global de production du PLA et, dans une bien moindre mesure, la réaction de formation d'oligoesters décrite ci-dessus engendre une certaine racémisation du PLA ce qui signifie l'apparition d'énantiomères de type D lorsque l'on travaille avec du PLA de type L (forme essentiellement présente sur le marché). De par la purification stéréospécifique du lactide dans le cadre de la présente invention, nous obtenons un appauvrissement en énantiomère non désiré contrairement aux procédés de recyclage existant.

### Description détaillée de l'invention

Le procédé de l'invention comprend une étape de mise en suspension du mélange de polymères dans un ester lactique apte à dissoudre la fraction PLA suivie d'une séparation, d'une part, de l'ester lactique, du PLA et des autres impuretés dissoutes et, d'autre part, du mélange des autres polymères et impuretés non solubles. La solution contenant le PLA ainsi obtenue est ensuite soumise à une réaction catalytique de dépolymérisation par transestérification afin de former des oligoesters. La réaction de dépolymérisation par transestérification est ensuite stoppée à un moment déterminé et l'ester lactique résiduel séparé.

L'oligoester ainsi obtenu subit ensuite une réaction de cyclisation afin de produire du lactide qui sera finalement purifié stéréospécifiquement de sorte à obtenir une fraction de lactide purifiée ayant une teneur en méso-lactide comprise entre 0,1 et 40 %.

Dans le cadre de la présente invention, les matières premières utilisées lors de ce recyclage chimique peuvent provenir de produits hors spécification dans les unités de production, de chutes de production dans les unités de transformation ainsi que de produits finis en fin de vie. Un broyage du mélange de polymères contenant du PLA peut être réalisé par diverses techniques, comme par exemple le broyage par cisaillement, par impact, à sec ou sous eau. L'objectif de cette étape étant d'augmenter la surface spécifique des matériaux, de manière à obtenir un rapport poids/volume compris entre 0,05 et 1,4 t/m3, ce qui permet de faciliter les étapes de manutention et d'accélérer l'étape suivante de dissolution, rendant le procédé plus facilement industrialisable. Dans le cadre de l'invention, une ou plusieurs étapes de broyage peuvent être envisagées, leur nombre étant fonction du produit de départ mais également du coût de ces opérations et de la granulation finale visée. Il est également possible de pré ou post traiter ces flux de mélange de polymères contenant du PLA notamment en procédant à un lavage à l'eau ou autres solutions tels que par exemple solution de soude, potasse, détergente, ... D'autres traitements, comme un tri manuel ou encore une séparation automatique (magnétique par exemple) peuvent être envisagés, tout cela dans le but d'éliminer d'éventuels déchets qui pourraient altérer la qualité du produit final ou en compliquer la purification. Il est également évident que si les déchets de mélange de polymères contenant du PLA à traiter ont une surface spécifique adéquate pour démarrer la mise en solution, on peut supprimer cette étape de broyage sans se départir du procédé de la présente invention.
Simultanément ou suite à cette étape de broyage, lorsqu'elle est réalisée, une étape de densification ou de compactage peut être envisagée afin de compacter la matière, ce qui améliorerait les étapes de manutention et de logistique.

Le mélange de polymères contenant du PLA, broyé ou non et compacté ou non, est ensuite mis en solution dans un ester lactique avant l'étape de dépolymérisation par transestérification. Il s'agit d'esters tels que du lactate de méthyle, du lactate d'éthyle, du lactate de propyle, du lactate de butyle, du lactate d'hexyle, ... et plus généralement d'un ester d'alkyle d'acide lactique dont le radical alkyle a de 1 à 12 atomes de carbones.
La mise en solution peut également être réalisée sans un broyage préalable si la forme du mélange de polymères contenant du PLA (rapport poids/volume) le permet. En effet, l'une des problématiques du traitement de ce type de flux est la différence de masse spécifique des différents matériaux retraités et ce même après l'étape de broyage. Cette mise en solution est assez rapide et peut être effectuée en quelques minutes.
Cette dissolution peut être préalable ou simultanée à l'étape suivante et réalisée à différentes températures allant jusqu'à la température de fusion du PLA. La société demanderesse a également mis en évidence qu'il était possible d'éliminer l'eau présente dans le PLA durant cette étape de mise en solution. En effet, compte tenu de la température d'ébullition des esters d'acide lactique préconisés dans le procédé de la présente invention, la mise en solution peut être effectuée à une température de plus de 100 °C et à pression atmosphérique et l'eau peut être facilement éliminée par condensation.

Dans le cas d'une contamination du flux de PLA par un autre polymère (PET, PE, PVC, PP, PC ou tout autre polymère courant), il est possible d'éliminer ce dernier, par exemple, par filtration si nécessaire à chaud. En effet, les esters lactiques ne permettent pas la mise en solution des polymères précités pour les temps de traitement requis. Cette séparation peut être effectuée avant ou après l'étape suivante.
La société demanderesse a montré que la dépolymérisation partielle du PLA, dans un rapport pondéral PLA/ester lactique compris entre 0,5 et 3, pouvait se faire par dépolymérisation par transestérification à une température comprise entre 80 et 180°C, préférentiellement entre 110 et 160°C et plus préférentiellement entre 120 et la température d'ébullition de l'ester lactique , sous dépression ou une pression comprise entre la pression atmosphérique et 10 bars voire plus. Cette étape de dépolymérisation par transestérification du PLA permet la production d'oligoesters par réaction d'un lien ester du PLA et d'une fonction alcool. De manière surprenante, la société demanderesse a trouvé que cette fonction alcool pouvait soit provenir d'un alcool ajouté soit de l'ester lactique ayant servi lors de la mise en solution. Dans le cadre de cette invention, peuvent être utilisés outre les esters lactiques cités précédemment, les alcools contenant 1 à 12 carbones, tels que le méthanol, l'éthanol, le n-butanol, l'isobutanol, le sec-butanol, le tert-butanol, le n-propanol, l'isopropanol, le 2-éthylhexanol, le 2-éthylbutanol, l'hexanol,... L'utilisation d'un catalyseur de transestérification s'avère nécessaire afin de déplacer l'équilibre de la réaction vers la formation de l'oligoester, ce catalyseur peut être solide ou liquide et de type acide de Lewis comme par exemple l'octoate d'étain, le lactate d'étain, l'octoate d'antimoine, l'octoate de zinc, l'APTS (acide para-toluène sulfonique), etc. ou préférentiellement basique, de la famille des Guanidines, comme par exemple le TBD (triazabicyclodécène) et ses dérivés.
Un mode particulier de cette invention est la mise en solution dans l'ester lactique durant laquelle l'humidité potentiellement présente dans le PLA est éliminée par évaporation. La libération d'acide lactique et l'oligomérisation catalysée par cette même molécule sont ainsi évitées. De même, lorsque la réaction de dépolymérisation par transestérification est menée en présence d'un catalyseur basique, l'absence d'eau du milieu permet d'éviter tout problème lié à l'acidité lors de cette étape et des étapes ultérieures du procédé.

Lorsque la masse moléculaire souhaitée est atteinte, entre 400 et 5000 uma, (mesuré par GPC avec calibration PS) préférablement entre 400 et 3000 uma, qualité qui permet d'éviter d'une part les problèmes liés au transfert de produits très visqueux et d'autre part une acidité résiduelle (en cas de présence d'eau) trop importante dans le produit obtenu à l'issue de l'étape de dépolymérisation, la réaction de dépolymérisation par transestérification est arrêtée. La réaction peut être stoppée par élimination de la fonction alcool (provenant de l'ester lactique ou de l'alcool), ce qui permet de garder le contrôle de la masse moléculaire de l'oligoester. Dans ce cadre, toute technologie permettant une extraction rapide de l'alcool ou de l'ester lactique sera privilégiée comme par exemple la technologie de la couche mince. La dépolymérisation peut également être stoppée par une neutralisation du catalyseur, ce qui permet d'être moins tributaire du facteur temps. Une autre possibilité consiste en un arrêt anticipé de la réaction sachant qu'elle se poursuivrait quelque peu lors d'un traitement plus lent d'élimination de l'ester lactique résiduel tel que par exemple une distillation batch. Une des options privilégiées de la présente invention consistera à éliminer l'alcool ou l'ester, ce qui n'exclut pas la possibilité de le maintenir dans le milieu et de passer directement à l'étape de cyclisation.
Une fois formé, il est préférable que l'oligoester soit directement traité, surtout si le catalyseur de transestérification n'a pas été désactivé. Cette étape consiste en la cyclisation catalytique et thermique de l'oligoester préférablement débarrassé de l'ester lactique, afin de produire une phase vapeur riche en lactide. L'utilisation d'un catalyseur s'avère indispensable afin de réduire la température de thermocraking et d'éviter ainsi la détérioration chimique et optique du lactide synthétisé. Le catalyseur sera solide ou liquide et de type acide de Lewis comme par exemple l'octoate d'étain, le lactate d'étain, l'octoate d'antimoine, l'octoate de zinc, l'APTS (acide para-toluène sulfonique), etc. ou préférentiellement basique, de la famille des Guanidines, comme par exemple le TBD (triazabicyclodécène) et ses dérivés. Idéalement, il sera identique à celui utilisé dans l'étape de dépolymérisation par transestérification.
Le réacteur sera préférablement sélectionné de sorte à maintenir le mélange (oligoesters/catalyseur) le moins longtemps possible (de 0 à 30 min et préférentiellement de 0 à 15 min) à la température de réaction tout en offrant une surface d'échange et un volume d'extraction important. La température de travail sera suffisante pour initier la réaction, mais pas trop élevée afin d'éviter une dégradation ou une racémisation du lactide : la température sera comprise entre 180 et 280°C. L'optimum de température sera fonction de la nature des oligoesters de départ (la masse moléculaire allant de 400 à 5000 uma), de la nature du catalyseur et de la pression dans le système.
Etant donné l'instabilité chimique du lactide aux températures de travail et afin de déplacer l'équilibre de la réaction vers la formation du lactide, il est important qu'il soit extrait le plus rapidement possible du milieu réactionnel. Dans ce cadre, il est préférable de maintenir le milieu réactionnel sous flux gazeux et/ou sous vide. La seconde option sera préférée car elle permet également de réduire la température de réaction. Suite aux différentes contraintes énoncées ci-avant et sans limiter la portée de la présente invention, l'utilisation d'un évaporateur de type couche mince semble particulièrement indiquée. En effet de ce type d'appareil, on extrait en pied un résidu liquide composé des oligoesters de hautes masses moléculaires. En tête, la phase vapeur riche en lactide est directement extraite et sélectivement condensée au niveau d'un condenseur fixé à une température bien déterminée. En effet, le condenseur est maintenu à une température telle que d'une part, les composés volatils comme l'eau, l'ester et l'alcool, restent en phase vapeur (alors que le lactide et les composés lourds sont condensés), et pas trop basse d'autre part pour éviter une cristallisation du lactide. En fonction de la nature et de la pureté du produit récolté (brut de lactide), cette température sera comprise entre 70 et 125°C. Il est également possible de travailler à plus basse température et ainsi cristalliser le lactide ce qui nécessitera l'utilisation d'un condenseur de type à surface raclée.
Etant donné que la matière de départ consiste en des oligoesters, le crude issu de cette condensation sélective sera plus stable, car moins réactif, dans le temps qu'un crude qui serait généré au départ d'acide lactique. De plus, ce crude aura une viscosité moindre, ce qui augmentera l'efficacité de certaines technologies de purification.

L'étape ultérieure du procédé consiste en une purification du crude qui permet de contrôler la teneur en méso-lactide dans le lactide final, cette teneur devant être comprise entre 0,1 et 40%, préférablement entre 0,1 et 20% ce qui permet de contrôler et donc d'éviter l'enrichissement en énantiomère D dans l'ensemble du processus.

Dans le cadre de l'invention, différents types de purifications sont envisageables, comme par exemple la cristallisation en milieu fondu, le méso-lactide ayant un point de fusion fort différent de celui du L-lactide, que cela soit en couche (type Sulzer) ou en suspension (type NIRO), la distillation avec contrôle de la teneur en méso-lactide en jouant sur le nombre d'étages théoriques et sur le taux de reflux, la cristallisation en solvant, l'extraction aqueuse (le méso-lactide étant plus sensible à l'hydrolyse que le L-lactide), ou en solvant (solubilité et température de cristallisation différente du méso-lactide par rapport au L-lactide). Toute autre technique permettant de remplir les conditions énoncées ci-avant est également valable dans le cadre de l'invention.
Le lactide ainsi obtenu aura une pureté élevée et la variabilité contrôlée en fonction du domaine d'application auquel il sera destiné.
En effet, s'il est destiné à la synthèse d'homo ou copolymères de PLA, son acidité résiduelle et sa teneur en eau résiduelle par exemple seront très basses, à savoir respectivement inférieure à 10 meq/kg pour la teneur en acidité résiduelle et inférieure à 100 ppm voir 50 ppm pour la teneur en eau résiduelle. La teneur en méso-lactide sera quant à elle variable en fonction des caractéristiques souhaitées pour le polymère (plus cristallin ou amorphe).
Par contre s'il est destiné à être utilisé comme intermédiaire réactionnel ou hydrolysé en acide lactique ou un de ses dérivés dans le cadre d'applications de type industrielle, la teneur en acidité, en eau résiduelle voire même en méso-lactide sera moins critique.

D'autres détails et particularités de l'invention, donnés ci-après à titre d'exemples non-limitatifs, ressortent de la description comme quelques formes possibles de sa réalisation.

### Exemple 1

1,00 kg de PLA (à 99,5 % en L(+)) est mis en solution dans 666 g de lactate d'éthyle. La mise en solution a été réalisée dans un ballon de 21 à pression atmosphérique et à une température de 140°C sous agitation. Lorsque tout le PLA a été mis en solution, 1 g de TBD est ajouté. La réaction de dépolymérisation par transestérification est poursuivie durant 24h à une température de 120°C sous reflux. La masse moléculaire moyenne en nombre étant de 1800 uma (mesurée par GPC). Le lactate d'éthyle est ensuite éliminé sur couche mince (130°C, 100 mbars). 1088 g d'oligoesters à 98,9 % en L(+) sont ainsi récupérés.
La réaction de cyclisation est réalisée à 250°C et une pression comprise entre 10 et 20 mbars en ajoutant 2% d'octoate d'étain au mélange d'oligoesters.
La vapeur générée (lactide impur - crude) est condensée et le crude obtenu purifié par melt cristallisation.
Le brut de lactide (768 g) obtenu est introduit dans un cristallisoir constitué par un tube vertical en inox. La double enveloppe du tube est alimentée en fluide caloporteur par un groupe de chauffe thermostatisé pour le contrôle des phases de cristallisation, de suage ou de refonte. Ce brut est fondu à 102°C. Ensuite, la cristallisation est initiée sur la paroi par une diminution progressive de la température. Une partie du brut est cristallisé sur les parois, alors que la partie centrale renferme la phase liquide (drain) contenant la majorité des impuretés. Lorsque la température atteint 60°C, la phase liquide est extraite par gravité et soufflage d'azote. Les cristaux sont encore recouverts d'un film d'impuretés que l'étape de suage doit éliminer, la surface du tube va dès lors être très progressivement chauffée de façon à faire fondre la surface des cristaux de moindre pureté. Le produit est finalement mené à sa température de fusion afin de le liquéfier et de le récolter par gravité.
Trois étages successifs de cristallisation ont été réalisés et les caractéristiques du produit final sont reprises dans le tableau 1.

**Tableau 1: Caractéristiques du produit final (lactide)**

| Eau ^{(a)} (%) | Acide lactique ^{(b)} (%) | Meso-LD ^{(c)} (%) | L-LD ^{(c)} (%) |
|---|---|---|---|
| 0,09 | 0,09 | 0,25 | 99,56 |

| | | | |
|---|---|---|---|
| (a) : déterminé par mesure Karl Fischer (b) : déterminé par titrage (c) : déterminés par GC | | | |

### Exemple 2

1,00 kg de PLA (à 95.3 % en L(+)) est mis en solution dans 666 g de lactate d'éthyle. La mise en solution a été réalisée dans un ballon de 21 à pression atmosphérique et à une température de 140°C sous agitation. Lorsque tout le PLA a été mis en solution, 319 g d'éthanol anhydre ainsi que 1 g d'octoate d'étain sont ajoutés. La réaction de dépolymérisation par transestérification est poursuivie durant 30h à une température de 120°C et dans un réacteur prouvant travailler sous pression. Le lactate d'éthyle et l'éthanol sont ensuite éliminés sur couche mince (130°C, 100 mbars). Un oligoester à 93.2% en L(+) est ainsi récupérés.
La réaction de cyclisation est réalisée à 250°C et une pression comprise entre 10 et 20 mbars en ajoutant 2% d'octate d'étain au mélange d'oligoesters.
La vapeur générée (lactide impur - crude) est condensée et une fraction du crude obtenu purifié par extraction en solvant.
Le brut de lactide (100 g) obtenu est chauffé à 85°C et mélangé à 100 g d'IPE (isopropyl éther) sous agitation jusqu'à homogénéisation. La suspension est alors progressivement refroidie jusque -18°C. Les cristaux sont ensuite filtrés et lavés à l'IPE. L'opération d'extraction a été répétée à deux puis trois reprises et le lactide obtenu est finalement séché sous vide sur évaporateur rotatif.
Les caractéristiques du produit final sont reprises dans le tableau 2.

**Tableau 2: Caractéristiques du produit final (lactide)**

| Nbre Extraction | Eau ^{(a)} (%) | Acide lactique ^{(b)} (%) | Meso-LD ^{(c)} (%) | L-LD ^{(c)} (%) |
|---|---|---|---|---|
| 2 | 0.012 | 0.12 | 3.7 | 96.2 |
| 3 | 0,012 | 0,1 | 1.3 | 98.5 |

| | | | | |
|---|---|---|---|---|
| (a) : déterminé par mesure Karl Fischer (b) : déterminé par titrage (c) : déterminés par GC | | | | |

### Exemple 3

Une petite quantité (10 g) du lactide produit dans l'exemple 1 est introduite dans un tube à essai sous balayage d'azote. Après fusion du produit (100°C), 6 mg d'octoate d'étain ont été ajouté (de sorte à respecter un rapport molaire produit/catalyseur de 4500). Une fois la solution homogénéisée, elle est plongée dans un bain d'huile dont la température est thermostatisée à 180°C. Après une heure de synthèse, le polymère est récupéré. Ce polymère a été analysé par GPC dans le chloroforme à 35°C : sa distribution de masses moléculaires pondérée en poids est de 98.000 (Mw avec une calibration PS).

### Exemple 4

Sur le lactide produit dans l'exemple 1, 100 g sont introduits dans un ballon. Afin d'obtenir un acide lactique concentré à 90%, 38,8 g d'eau déminéralisée sont ajoutés. La solution est alors chauffée à 100°C durant 3h.
Les caractéristiques de l'acide lactique final sont reprises dans le tableau 3.

**Tableau 3: Caractéristiques de l'acide lactique**

| Pureté stéréospécifique ^{(a)} (%) | Concentration ^{(b)} (%) | Coloration FC^{(c)} (Hz) | Coloration HS ^{(d)} (Hz) | Teneur en Cations -Métaux ^{(e)} |
|---|---|---|---|---|
| 99,8 | 90,5 | 8 | 20 | 23 ppm |

| | | | | |
|---|---|---|---|---|
| (a) : déterminé par dosage enzymatique (b) : déterminé par titrage (c) : Fresh Color déterminé par colorimétrie (d) : Heat Stability déterminé par colorimétrie après chauffe 2h à reflux (e) : déterminée par spectrométrie | | | | |

### Exemple 5

800 g de PLA (à 95,5 % en L(+)) sont mis en solution dans 533 g de lactate d'éthyle. La mise en solution a été réalisée dans un réacteur de 2 1 permettant de travailler sous pression et à une température de 140°C sous agitation (cette opération a été menée à pression atmosphérique). Lorsque tout le PLA a été mis en solution, 51 g d'éthanol et 0,8 g de TBD sont ajoutés. La réaction de dépolymérisation par transestérification est poursuivie durant 24h à une température de 140°C. La masse moléculaire moyenne en nombre étant de 1800 uma (mesurée par GPC). Le lactate d'éthyle et l'éthanol sont ensuite éliminés sur couche mince (140°C, 100 mbars). 1060 g d'oligoesters à 95,2 % en L(+) sont ainsi récupérés.
Une procédure identique à celle décrit dans l'exemple 1 a été suivie et à l'issue des trois étage de cristallisation, le produit dont les caractéristiques sont reprises dans le tableau 4 ci-dessous a été obtenu.

**Tableau 4: Caractéristiques du produit final (lactide)**

| Eau ^{(a)} (%) | Acide lactique ^{(b)} (%) | Meso-LD ^{(c)} (%) | L-LD ^{(c)} (%) |
|---|---|---|---|
| 0,032 | 0,024 | 0 | 99,89 |

| | | | |
|---|---|---|---|
| (a) : déterminé par mesure Karl Fischer (b) : déterminé par titrage (c) : déterminés par GC | | | |

## Revendications

1. Procédé de recyclage chimique et stéréospécifique d'un mélange de polymères contenant de l'acide polylactique PLA **caractérisé en ce qu'**il comprend :
a.une mise en solution du mélange de polymères dans un ester lactique apte à dissoudre la fraction PLA ;
b. une dépolymérisation par transestérification de la fraction de PLA provenant de l'étape précédente, cette dépolymérisation étant arrêtée lorsque le mélange d'oligoesters provenant de la dépolymérisation atteint une masse moléculaire moyenne en nombre comprise entre 400 et 5.000 uma mesuré par GPC:

2. Procédé selon la revendication 1 **caractérisé en ce qu'**entre les étapes a et b, il comprend une étape de séparation de l'ester lactique et du PLA dissous d'une part, et du mélange de polymères non solubles d'autre part.

3. Procédé selon la revendication 1 ou 2 **caractérisé en ce que** le mélange d'oligoesters de l'étape b est soumis a une étape de cyclisation catalytique avec production d'une phase vapeur riche en lactide et une phase liquide riche en oligoesters.

4. Procédé selon l'une quelconque des revendications 1 à 3 **caractérisé en ce que** la phase vapeur riche en lactide est condensée sélectivement sous forme d'un brut de lactide débarrassé des composants volatils.

5. Procédé selon l'une quelconque des revendications 1 à 4 **caractérisé en ce que** le brut de lactide est soumis à une purification stéréospécifique en une fraction de lactide purifié avec une teneur en méso-lactide comprise entre 0,1 et 40 % en poids.

6. Procédé selon l'une quelconque des revendications 1 à 5 **caractérisé en ce que** la mise en solution de l'étape a est effectuée avec un rapport pondéral PLA/ester lactique compris entre 0,5 et 3.

7. Procédé selon l'une quelconque des revendications 1 à 6 **caractérisé en ce que** le mélange de polymères broyé et compacté présente avant sa mise en solution dans l'ester lactique un rapport poids/volume compris entre 0,005 et 1,4 t / m³ .

8. Procédé selon l'une quelconque des revendications 1 à 7 **caractérisé en ce que** la fonction alcool impliquée dans la réaction de dépolymérisation par transestérification provient de l'ester lactique ayant servi à la mise en solution.

9. Procédé selon l'une quelconque des revendications 1 à 7 **caractérisé en ce que** la fonction alcool impliquée dans la réaction de dépolymérisation par transestérification provient d'un alcool ajouté.

10. Procédé selon l'une quelconque des revendications 1 à 9 **caractérisé en ce que** l'on conduit la réaction de dépolymérisation par transestérification en présence d'un catalyseur.

11. Procédé selon l'une quelconque des revendications 1 à 9 **caractérisé en ce que** la réaction de dépolymérisation par transestérification est stoppée par élimination de la fonction alcool.

12. Procédé selon l'une quelconque des revendications 1 à 10 **caractérisé en ce que** la réaction de dépolymérisation par transestérification est stoppée par neutralisation du catalyseur.

13. Procédé selon l'une quelconque des revendications 1 à 12 **caractérisé en ce que** l'ester lactique résiduel est séparé des oligoesters avant d'entamer l'étape de cyclisation.

14. Procédé selon l'une quelconque des revendications 1 à 13 **caractérisé en ce que** le catalyseur de dépolymérisation par transestérification est préférentiellement identique au catalyseur de cyclisation.

15. Procédé selon l'une quelconque des revendications 1 à 14 **caractérisé en ce que** le brut de lactide est purifié au moyen d'une méthode stéréospécifique choisie parmi la cristallisation en milieu fondu en couche, la cristallisation en milieu fondu en suspension, la cristallisation en milieu solvant, la distillation, l'extraction aqueuse.

## Patentansprüche

1. Verfahren für chemisches und stereospezifisches Recycling einer Polymermischung, die PLA-Polymilchsäure enthält, **dadurch gekennzeichnet, dass** es umfasst:
a. eine Auflösung der Polymermischung in einem Milchsäureester, der imstande ist, die PLA-Fraktion aufzulösen,
b. eine Depolymerisation durch Umesterung der PLA-Fraktion aus vorherigem Schritt, wobei diese Depolymerisation gestoppt wird, wenn das Oligoestergemisch im Ergebnis der Depolymerisation eine zahlenmäßig durchschnittliche molekulare Masse zwischen 400 und 5.000 uma inklusive, gemessen durch GPC, erreicht.

2. Verfahren nach Anspruch 1, **dadurch gekennzeichnet, dass** es zwischen den Schritten a und b einen Separationsschritt des Milchsäureesters und der gelösten PLA einerseits und der unlöslichen Polymermischung andererseits umfasst.

3. Verfahren nach Anspruch 1 oder 2, **dadurch gekennzeichnet, dass** das Oligoestergemisch von Schritt b einem Schritt der katalytischen Cyclisierung mit Produktion einer lactidreichen Dampfphase und einer oligoesterreichen flüssigen Phase unterzogen wird.

4. Verfahren nach einem der Ansprüche 1 bis 3, **dadurch gekennzeichnet, dass** die lactidreiche Dampfphase selektiv in Form eines von flüchtigen Komponenten befreiten Lactidrohprodukts kondensiert wird.

5. Verfahren nach einem der Ansprüche 1 bis 4, **dadurch gekennzeichnet, dass** das Lactidrohprodukt einer stereospezifischen Reinigung in eine gereinigte Lactidfraktion mit einem Mesolactidgehalt zwischen 0,1 und 40 Gew.-% inklusive unterzogen wird.

6. Verfahren nach einem der Ansprüche 1 bis 5, **dadurch gekennzeichnet, dass** die Auflösung von Schritt a mit einem Gewichtsverhältnis PLA/Milchsäureester zwischen 0,5 und 3 inklusive durchgeführt wird.

7. Verfahren nach einem der Ansprüche 1 bis 6, **dadurch gekennzeichnet, dass** die gemahlene und kompaktierte Polymermischung vor ihrer Auflösung im Milchsäureester ein Gewichts/Volumenverhältnis zwischen 0,005 und 1,4 t/m³ inklusive aufweist.

8. Verfahren nach einem der Ansprüche 1 bis 7, **dadurch gekennzeichnet, dass** die Alkoholfunktion, die an der Depolymerisationsreaktion durch Umesterung beteiligt ist, von dem Milchsäureester stammt, der zur Auflösung gedient hatte.

9. Verfahren nach einem der Ansprüche 1 bis 7, **dadurch gekennzeichnet, dass** die Alkoholfunktion, die an der Depolymerisationsreaktion durch Umesterung beteiligt ist, von einem hinzugefügten Alkohol stammt.

10. Verfahren nach einem der Ansprüche 1 bis 9, **dadurch gekennzeichnet, dass** die Depolymerisationsreaktion durch Umesterung in Anwesenheit eines Katalysators durchgeführt wird.

11. Verfahren nach einem der Ansprüche 1 bis 9, **dadurch gekennzeichnet, dass** die Depolymerisationsreaktion durch Umesterung durch Entfernen der Alkoholfunktion gestoppt wird.

12. Verfahren nach einem der Ansprüche 1 bis 10, **dadurch gekennzeichnet, dass** die Depolymerisationsreaktion durch Umesterung durch Neutralisieren des Katalysators gestoppt wird.

13. Verfahren nach einem der Ansprüche 1 bis 12, **dadurch gekennzeichnet, dass** der restliche Milchsäureester vor Beginn des Schritts der Cyclisierung von den Oligoestern separiert wird.

14. Verfahren nach einem der Ansprüche 1 bis 13, **dadurch gekennzeichnet, dass** der Depolymerisationskatalysator durch Umesterung vorzugsweise mit dem Cyclisierungskatalysator identisch ist.

15. Verfahren nach einem der Ansprüche 1 bis 14, **dadurch gekennzeichnet, dass** das Lactidrohprodukt mit Hilfe einer stereospezifischen Methode gereinigt wird, die aus der Schichtschmelzkristallisation, der Suspensionsschmelzkristallisation, der Lösungsmittelkristallisation, der Destillation, der wässrigen Extraktion ausgewählt ist.

## Claims

1. A method for chemical and stereochemical recycling a mixture of polymers containing polylactic acid PLA **characterized in that** it comprises:
a. putting into solution the mixture of polymers in a lactic ester able to dissolve the PLA fraction;
b. depolymerizing by transesterification the PLA fraction from the previous step, this depolymerization being stopped when the mixture of oligoesters from the depolymerization attains a number average molecular mass comprised between 400 and 5,000 amu measured by GPC.

2. The method according to claim 1, **characterized in that** between the steps a and b, it comprises a step for separating the lactic ester and the dissolved PLA on the one hand, and of the mixture of non-soluble polymers on the other hand.

3. The method according to claim 1 or 2, **characterized in that** the mixture of oligoesters of step b is subject to a catalytic cyclization step with production of a lactide-rich vapor phase and a liquid phase rich in a oligoesters.

4. The method according to any of claims 1 to 3, **characterized in that** the lactide-rich vapor phase is selectively condensed in the form of crude lactide free of volatile components.

5. The method according to any of claims 1 to 4, **characterized in that** the crude lactide is subject to stereospecific purification into a purified lactide fraction with a meso-lactide content comprised between 0.1 and 40% by weight.

6. The method according to any of claims 1 to 5, **characterized in that** the putting into solution of step a is carried out with a PLA/lactic ester weight ratio comprised between 0.5 and 3.

7. The method according to any of claims 1 to 6, **characterized in that** the milled and compacted mixture of polymers has, before its putting into solution in the lactic ester, a weight/volume ratio comprised between 0.005 and 1.4 t/m³.

8. The method according to any of claims 1 to 7, **characterized in that** the alcohol function involved in the depolymerization reaction by transesterification stems from the lactic ester having been used during the putting into solution.

9. The method according to any of claims 1 to 7, **characterized in that** the alcohol function involved in the depolymerization reaction by transesterification stems from an added alcohol.

10. The method according to any of claims 1 to 9, **characterized in that** the depolymerization reaction by transesterification is conducted in the presence of a catalyst.

11. The method according to any of claims 1 to 9, **characterized in that** the depolymerization reaction by transesterification is stopped by removing the alcohol function.

12. The method according to any of claims 1 to 10, **characterized in that** the depolymerization reaction by transesterification is stopped by neutralizing the catalyst.

13. The method according to any of claims 1 to 12, **characterized in that** the residual lactic ester is separated from the oligoesters before starting the cyclization step.

14. The method according to any of claims 1 to 13, **characterized in that** the catalyst for the depolymerization reaction by transesterification is preferentially identical with the cyclization catalyst.

15. The method according to any of claims 1 to 14, **characterized in that** the crude lactide is purified by means of a stereospecific method selected from layer-based melt crystallization, suspension-based melt crystallization, crystallization in a solvent medium, distillation, extraction with water.
